(19) Europäisches Patentamt
European Patent Office
Office européen des brevets

(11) Numéro de publication : **0 356 412 B1**

(12) # FASCICULE DE BREVET EUROPEEN

(45) Date de publication du fascicule du brevet : **13.05.92 Bulletin 92/20**

(51) Int. Cl.$^5$ : **A61K 31/535**

(21) Numéro de dépôt : **89870119.8**

(22) Date de dépôt : **28.07.89**

(54) **Utilisation de 2-amino-4-morpholino-6-propyl-1,3,5-triazines pour la préparation de médicaments destinés au traitement destroubles de la cognition et de l'humeur.**

(30) Priorité : **16.08.88 GB 8819494**

(43) Date de publication de la demande : **28.02.90 Bulletin 90/09**

(45) Mention de la délivrance du brevet : **13.05.92 Bulletin 92/20**

(84) Etats contractants désignés : **AT BE CH DE ES FR GB GR IT LI LU NL SE**

(56) Documents cités :
**DE-A- 2 226 474**
**FR-A- 2 262 512**
**US-A- 3 549 755**
**YAKUGAKU ZASSHI, vol. 95, no. 5, 1975, pages 499-511; T. TSUJIKAWA et al.: "Studies on heterocyclic compounds. I. Synthesesof 1,3,5-triazine derivatives and their pharmacological activities"**

(56) Documents cités :
**BOLL. CHIM. FARM., vol. 116, 1977, pages 637-643; T. SCIORTINO et al.: "Nuove s-triazine sostituite di possibile interessefamacologico"**
**Dictionnaire français de Médecine et de Biologie, Tome III, 1972, p.452**

(73) Titulaire : **UCB S.A.**
**Avenue Louise 326, Bte 7**
**B-1050 Bruxelles (BE)**

(72) Inventeur : **Wülfert, Ernst**
**52, Avenue des Aubépines**
**B-1180 Bruxelles (BE)**
Inventeur : **Gobert, Jean**
**120, rue du Cornet**
**B-1040 Bruxelles (BE)**
Inventeur : **Cossement, Eric**
**105, rue des Echevins**
**B-1050 Bruxelles (BE)**

(74) Mandataire : **Dusseldorp, Raymond et al**
**U.C.B. S.A. Département D.T.B. 326, avenue Louise, Bte 7**
**B-1050 Bruxelles (BE)**

EP 0 356 412 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention se rapporte à l'utilisation de 2-amino-4-morpholino-6-propyl-1,3,5-triazines ainsi que de leurs sels d'addition d'acides non toxiques pharmaceutiquement acceptables pour la préparation de médicaments destinés au traitement des troubles cognitifs associés à l'âge et aux syndromes démentiels.

Selon la demande de brevet japonaise 69688/74, on connaît déjà des 2-amino-1,3,5-triazines substituées en position 4 par un groupe amino, amino mono- ou disubstitué par un radical alkyle, alkényle, cycloalkyle, aryle ou aralkyle, ou par un radical hétérocyclique azoté, et en position 6 par un radical alkyle, aryle ou aralkyle. Un composé représentatif de cette famille est la 2-amino-4-morpholino-6-propyl-1,3,5-triazine. Selon cette demande de brevet, ces composés possèdent la propriété d'augmenter la sécrétion des corticoïdes et en particulier des glucocorticoïdes.

Par ailleurs, K. WAKABAYASHI KO et al., (Yuki Gosei Kagaku Kyokai Shi,28,(1970),252-260; Chem.Abstr.72,(1970),100653v) ont étudié l'activité herbicide de 2-alkyl-4,6-bis(alkylamino)-1,3,5-triazines dans lesquelles le radical en position 2 contient de 1 à 17 atomes de carbone et peut aussi représenter le groupe trichlorométhyle, tribromométhyle ou 2-chloréthyle, tandis que les radicaux en positions 4 et 6 sont identiques et peuvent être substitués par un groupe hydroxyle, ou bien, représentent un hétérocycle azoté tel que le radical pipéridino ou morpholino. Un composé représentatif de cette famille est la 2-méthyl-4,6-dimorpholino-1,3,5-triazine. On notera toutefois que les propriétés pharmacologiques de ces composés n'ont pas été examinées par ces auteurs.

D'autre part, dans un article de T. SCIORTINO et al. (Boll.Chim. Farm.116,(1977),637-643) on étudie également des 2-amino-1,3,5-triazines substituées en position 4 par un radical diméthylamino, butylamino, phénéthylamino ou morpholino, et en position 6 par un radical alkyle contenant 5 à 17 atomes de carbone, pour leur activité antivirale, antibactérienne, antifongique, anticonvulsivante, hypotensive, antihypoxique, antiarythmique, fibrinolytique, antithrombotique, antiallergique, antihistaminique, immunologique, ainsi que pour leur activité dépressive ou stimulante d'origine centrale. Deux composés représentatifs de cette famille sont la 2-amino-4-morpholino-6-pentyl-1,3,5-triazine et la 2-amino-4-(diméthylamino)-6-pentyl-1,3,5-triazine. Toutefois, aucune activité pharmacologique intéressante n'a été décelée chez ces composés.

Enfin, selon la demande de brevet française 2.262.512, on connaît des 2-amino-1,3,5-triazines substituées en position 4 par un atome d'halogène, un groupe hydrazino, un radical dialkylaminoalkylamino ou un radical hétérocyclique azoté comme par exemple un radical pipérazino substitué, et en position 6 par un atome d'hydrogène, un radical alkyle ou phényle, le groupe amino en position 2 pouvant éventuellement être substitué par un radical alkyle ou dialkylaminoalkyle ou faire partie d'un cycle pipérazinique.

Selon cette demande de brevet française, ces composés sont utilisables en thérapeutique humaine ou vétérinaire notamment comme analgésiques et psychotropes. Toutefois, les essais pharmacologiques décrits fournissent seulement les résultats obtenus avec trois dérivés pipéraziniques et uniquement en ce qui concerne leur activité analgésique. Quant à l'activité psychotrope de ces produits, elle n'est ni précisée, ni examinée dans les essais.

La demanderesse a présentement découvert que des 2-amino-4-morpholino-6-propyl-1,3,5-triazines convenablement substituées possèdent la propriété avantageuse de potentialiser les effets cholinergiques centraux et périphériques provoqués par un agent cholinomimétique tel que, par exemple, l'oxotrémorine, alors que ces composés ne présentent pas d'effet cholinergique propre. De plus, elle a trouvé également que ces composés ont la propriété avantageuse d'atténuer les effets résultant d'un hypofonctionnement cholinergique induit par un antagoniste cholinergique tel que, par exemple, la scopolamine. Le système cholinergique est largement impliqué dans les phénomènes de mémorisation et d'apprentissage. Ainsi, par exemple, l'administration à des sujets jeunes d'un agent anticholinergique tel que la scopolamine fait apparaître des déficiences de la mémoire semblables à celles observées chez des sujets âgés. Inversement, des agents cholinergiques, comme la physostigmine, sont capables de combattre l'amnésie résultant de l'administration d'agents anticholinergiques (S.D. GLICK et al., Behavioral Biology,7,(1972),245-254; U. SCHINDLER et al., Drug Develop.Res. 4,(1984),567-576). Par ailleurs, il est actuellement bien établi qu'une des caractéristiques le plus fréquemment associées aux démences est précisément une dégradation du système cholinergique (Cellular and molecular basis of cholinergic function, Ed. M.J. DOWDALL & J.N. HAWTHORNE, 1987, Chapitre 99: art. de A. NORDBERG et al.). C'est pourquoi, ces composés sont très utiles pour la préparation de médicaments destinés au traitement des troubles cognitifs associés à l'âge et aux syndromes démentiels. En particulier, ils trouvent une application dans le traitement des troubles associés à la maladie d'Alzheimer, aux démences séniles de type Alzheimer et à toute pathologie cognitive évolutive (C.G. GOTTFRIES, Psychopharmacology,86, (1985),245-252; C.G. GOTTFRIES, Neurobiology of Ageing,4,(1983),261-271).

La présente invention a donc pour objet l'utilisation de 2-amino-4-morpholino-6-propyl-1,3,5-triazines répondant à la formule générale

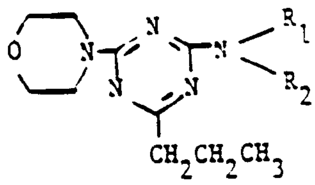

(I)

dans laquelle

$R_1$ représente un atome d'hydrogène, un radical alkyle, aralkyle ou acétyle,

$R_2$ représente un atome d'hydrogène, un groupe hydroxyle, un radical alkyle, hydroxyalkyle, alkoxyalkyle, dialkylamino, aryl-hydroxyalkyle, (hydroxy-cycloalkyl)alkyle, alkanoyloxyalkyle, benzoyloxyalkyle, phénylacétyloxyalkyle, aminocarbonyloxyalkyle, un groupe $COR_3$ dans lequel $R_3$ représente un radical alkyle, aryle, halogénoaryle, alkylaryle, alkoxyaryle, aralkyle ou aryloxy ou un groupe $CONR_4R_5$ dans lequel $R_4$ et $R_5$ représentent de l'hydrogène ou un radical alkyle, ou

$R_1$ et $R_2$ forment conjointement avec l'atome d'azote auquel ils sont attachés un radical alkylènimino substitué par un radical hydroxyalkyle, les radicaux alkyle, alkoxy et alkanoyloxy ayant de 1 à 4 atomes de carbone et les radicaux cycloalkyle et alkylènimino ayant de 4 à 6 atomes de carbone, avec la restriction que lorsque $R_1$ représente le radical acétyle, $R_2$ représente un radical acétoxyalkyle,

ainsi que de leurs sels d'addition d'acides non toxiques pharmaceutiquement acceptables, pour la préparation de médicaments destinés au traitement des troubles cognitifs associés à l'âge et aux syndromes démentiels.

Comme exemples de radical hydroxyalkyle, on citera les radicaux 2-hydroxyéthyle, 3-hydroxypropyle, 2-hydroxypropyle, 2-hydroxybutyle, 1-(hydroxyméthyl)propyle, 2,3-dihydroxypropyle, 2-hydroxy-1,1-bis(hydroxyméthyl)éthyle.

Comme exemple de radical aryl-hydroxyalkyle, on citera le radical 2-hydroxy-1-(hydroxyméthyl)-2-phényléthyle.

Comme exemple de radical alkanoyloxyalkyle, on citera les radicaux 2-(acétoxy)éthyle, 2-(isobutyryloxy)éthyle, etc.

Comme exemple de radical (hydroxy-cycloalkyl)alkyle, on citera le radical (1-hydroxycyclohexyl)méthyle, etc.

Comme exemple de radical alkylènimino substitué par un radical hydroxyalkyle, on citera les radicaux 2-hydroxyméthyl-pyrrolidino, 2-hydroxyméthyl-pipéridino.

Les 2-amino-4-morpholino-6-propyl-1,3,5-triazines préférées dont l'utilisation en thérapeutique constitue l'objet de la présente invention, sont celles répondant à la formule générale I dans laquelle

$R_1$ représente un atome d'hydrogène ou un radical alkyle,

$R_2$ représente un atome d'hydrogène, un groupe hydroxyle, un radical alkyle, hydroxyalkyle, alkoxyalkyle, dialkylamino, alkanoyloxyalkyle et un groupe $COR_3$ dans lequel $R_3$ représente un radical aryle substitué ou non par un atome d'halogène ou un radical alkyle ou alkoxy, les radicaux alkyle, alkoxy et alkanoyloxy ayant de 1 à 4 atomes de carbone,

ainsi que leurs sels d'addition d'acides non toxiques pharmaceutiquement acceptables.

Les composés particulièrement préférés à utiliser sont:

- le chlorhydrate de 2-amino-4-morpholino-6-propyl-1,3,5-triazine,
- le chlorhydrate de 2-(méthylamino)-4-morpholino-6-propyl-1,3,5-triazine,
- le 2-[(4-morpholino-6-propyl-1,3,5-triazin-2-yl)amino]-éthanol,
- la 2-(hydroxyamino)-4-morpholino-6-propyl-1,3,5-triazine,
- la 2-[(2-méthoxyéthyl)amino]-4-morpholino-6-propyl-1,3,5-triazine,
- le (S)-3-[(4-morpholino-6-propyl-1,3,5-triazin-2-yl)amino]-2-propanol,
- le (R)-3-[(4-morpholino-6-propyl-1,3,5-triazin-2-yl)amino]-2-propanol,
- la 2-(2,2-diméthylhydrazino)-4-morpholino-6-propyl-1,3,5-triazine,
- le chlorhydrate de N-(4-morpholino-6-propyl-1,3,5-triazin-2-yl)-benzamide,
- le chlorhydrate de N-méthyl-N-(4-morpholino-6-propyl-1,3,5-triazin-2-yl)-benzamide,
- la 2-[[2-(acétoxy)éthyl]amino]-4-morpholino-6-propyl-1,3,5-triazine.

La présente invention concerne également l'utilisation des sels d'addition d'acides non toxiques pharmaceutiquement acceptables des 2-amino-4-morpholino-6-propyl-1,3,5-triazines de formule I. Comme exemples d'acides pharmaceutiquement acceptables, on peut citer les acides minéraux comme les acides chlorhydrique, bromhydrique, sulfurique, nitrique, phosphorique, et les acides organiques comme les acides acétique, citrique, tartrique, benzoïque, salicylique, maléique.

Les composés de formule I peuvent se présenter soit sous la forme racémique soit sous forme de l'un ou l'autre énantiomère, lorsque la molécule comporte un atome de carbone asymétrique. L'utilisation ces diverses

formes entre également dans le cadre de la présente invention.

Les composés de formule I sont des composés nouveaux, à l'exception de celui dans lequel $R_1$ et $R_2$ représentent un atome d'hydrogène (cf. demande japonaise 69688/74 citée plus haut).

Les 2-amino-4-morpholino-6-propyl-1,3,5-triazines à utiliser, conformément à la présente invention, peuvent être préparées selon des méthodes conventionnelles et plus particulièrement par l'un ou l'autre des procédés suivants:

(a) Lorsque dans la formule I, $R_1$ représente un atome d'hydrogène, un radical alkyle ou aralkyle et $R_2$ représente un atome d'hydrogène, un groupe hydroxyle, un radical alkyle, hydroxyalkyle, alkoxyalkyle, dialkylamino, aryl-hydroxyalkyle ou (hydroxy-cycloalkyl)alkyle, ou $R_1$ et $R_2$ forment conjointement avec l'atome d'azote auquel ils sont attachés un radical alkylènimino substitué par un radical hydroxyalkyle, on fait réagir la 2-chloro-4-morpholino-6-propyl-1,3,5-triazine de formule II avec une amine de formule $HNR_1R_2$ (III) selon l'équation

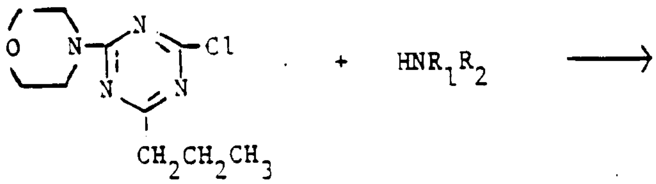

(I)

(II) (III)

dans ces formules $R_1$ et $R_2$ ont la signification donnée ci-dessus.

(b) Lorsque dans la formule I, $R_1$ représente un atome d'hydrogène, un radical alkyle ou aralkyle et $R_2$ représente un atome d'hydrogène, un groupe hydroxyle, un radical alkyle, hydroxyalkyle, alkoxyalkyle, dialkylamino, aryl-hydroxyalkyle ou (hydroxy-cycloalkyl)alkyle, ou $R_1$ et $R_2$ forment conjointement avec l'atome d'azote auquel ils sont attachés un radical alkylèneimino substitué par un radical hydroxyalkyle, on fait réagir une 2-amino-4-chloro-6-propyl-1,3,5-triazine de formule IV avec une morpholine de formule V, selon l'équation

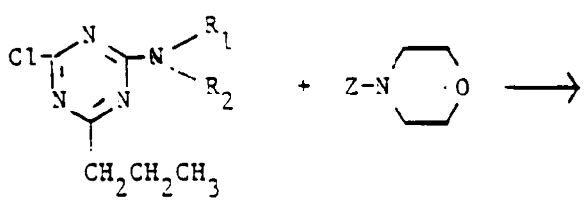
(I)

(IV) (V)

dans ces formules $R_1$ et $R_2$ ont la signification donnée ci-dessus et Z représente un atome d'hydrogène ou un radical méthyle.

Les procédés (a) et (b) sont réalisés à température élevée, généralement à la température d'ébullition du solvant employé, et en présence d'une base. Le solvant dans lequel on effectue ces réactions est soit l'amine elle-même, utilisée en excès, soit un solvant organique inerte, de préférence le dioxanne, et dans ce dernier cas, la base utilisée est une base minérale ou organique, autre que l'amine mise en oeuvre dans la réaction, par exemple la triéthylamine.

La 2-chloro-4-morpholino-6-propyl-1,3,5-triazine (II) utilisée comme produit de départ est déjà connue (T. TSUJIKAWA et al., Yakugaku Zasshi,95,(1975),512-520).

Les composés de départ de formule IV sont préparés, selon les méthodes conventionnelles, en faisant réagir la 2,4-dichloro-6-propyl-1,3,5-triazine avec une amine de formule $HNR_1R_2$ (III) dans laquelle $R_1$ et $R_2$ ont la signification donnée ci-dessus.

(c) Lorsque dans la formule I, $R_1$ représente un atome d'hydrogène, un radical alkyle ou aralkyle et $R_2$ représente le groupe $COR_3$ dans lequel $R_3$ est un radical alkyle, aryle, halogénoaryle, alkylaryle, alkoxyaryle, aralkyle ou aryloxy, les radicaux alkyles ayant de 1 à 4 atomes de carbone, on fait réagir dans des proportions équimolaires une 2-amino-4-morpholino-6-propyl-1,3,5-triazine de formule VI avec un halogénure de $R_3$-carbonyle de formule VII, selon l'équation

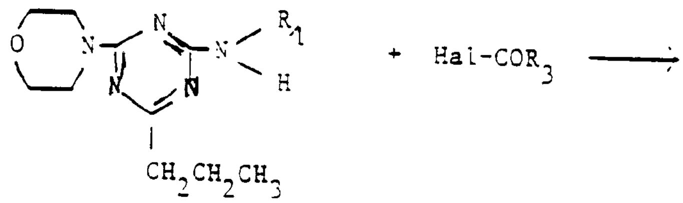


(I)

(VI) (VII)

dans ces formules $R_1$ et $R_3$ ont la signification donnée ci-dessus et Hal représente un atome d'halogène, de préférence un atome de chlore. Cette réaction, connue en soi, est généralement effectuée dans un solvant organique comme, par exemple, le dichlorométhane, le dichloroéthane ou la pyridine, à une température comprise entre la température ambiante et la température d'ébullition du solvant et en présence d'un accepteur d'acide tel qu'une base organique tertiaire (p.ex. la triéthylamine ou la pyridine) ou une base minérale.

Les composés de départ de formule VI peuvent être préparés selon l'un ou l'autre des deux procédés (a) ou (b) décrits précédemment.

(d) Lorsque dans la formule I, $R_1$ représente un atome d'hydrogène, un radical alkyle ou aralkyle et $R_2$ représente un radical alkanoyloxyalkyle, benzoyloxyalkyle ou phénylacétyloxyalkyle, les radicaux alkyle et alkanoyle ayant de 1 à 4 atomes de carbone, on fait réagir dans des proportions équimolaires, un (4-morpholino-6-propyl-1,3,5-triazin-2-yl)-aminoalcanol de formule VIII avec un halogénure de $R_6$-carbonyle de formule IX, selon l'équation

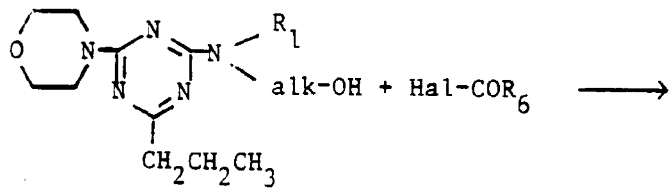


(I)

(VIII) (IX)

dans ces formules $R_1$ a la signification donnée ci-dessus, alk représente un radical alkylène en $C_1$-$C_4$, $R_6$ représente un radical alkyle en $C_1$-$C_4$, phényle ou benzyle et Hal représente un atome d'halogène, de préférence un atome de chlore.

Cette réaction, connue en soi, est généralement effectuée dans un solvant organique comme par exemple, le dichlorométhane, le dichloroéthane ou la pyridine à une température comprise entre 0°C et 25°C et en présence d'un accepteur d'acide tel qu'une base organique tertiaire (p.ex. la triéthylamine ou la pyridine) ou une base minérale.

Les composés de départ de formule VIII peuvent être préparés selon l'un ou l'autre des deux procédés (a) ou (b) décrits précédemment.

(e) Lorsque dans la formule I, $R_1$ représente un atome d'hydrogène, un radical alkyle ou aralkyle et $R_2$ un groupe $CONR_4R_6$ dans lequel $R_4$ et $R_6$ représentent de l'hydrogène ou un radical alkyle, les radicaux alkyles ayant de 1 à 4 atomes de carbone, on fait réagir un (4-morpholino-6-propyl-1,3,5-triazin-2-yl)carbamate de phényle de formule X avec un composé azoté de formule $HNR_4R_6$ (XI), selon l'équation

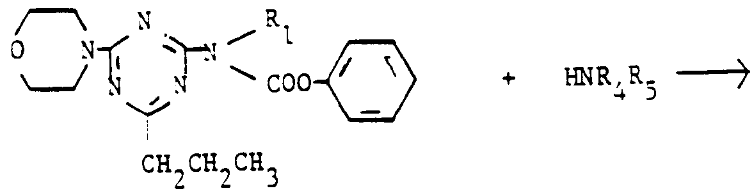

(I)

(X) (XI)

dans ces formules $R_1$, $R_4$ et $R_5$ ont la signification donnée ci-dessus. Cette réaction est généralement effectuée dans un solvant inerte tel que le dichlorométhane, et à une température comprise entre -45°C et +25°C.

Les composés de départ de formule X peuvent être préparés selon le procédé (c) ci-dessus, en faisant réagir une 2-amino-4-morpholino-6-propyl-1,3,5-triazine de formule VI, dans laquelle $R_1$ a la signification donnée ci-dessus, avec un halogénure de $R_3$-carbonyle de formule VII dans laquelle $R_3$ représente le radical phénoxy et Hal représente un atome d'halogène, de préférence un atome de chlore.

(f) Lorsque dans la formule I, $R_1$ représente un atome d'hydrogène, un radical alkyle ou aralkyle, et $R_2$ un radical aminocarbonyloxyalkyle, les radicaux alkyles ayant de 1 à 4 atomes de carbone, on fait réagir de l'ammoniac avec une 2-morpholino-4-(phénoxycarbonyloxyalkylamino)-6-propyl-1,3,5-triazine de formule XII, selon l'équation

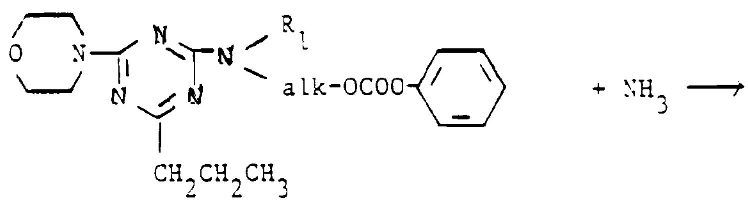

(I)

(XII)

dans ces formules $R_1$ a la signification donnée ci-dessus et alk représente un radical alkylène en $C_1$-$C_4$. Cette réaction est généralement effectuée dans un solvant inerte tel que le dichlorométhane, et à une température comprise entre -35°C et -45°C.

Les composés de départ de formule XII peuvent être préparés selon le procédé (d) ci-dessus, en faisant réagir un (4-morpholino-6-propyl-1,3,5-triazin-2-yl)aminoalcanol de formule VIII avec un halogénoformiate de phényle de formule XIII, selon l'équation

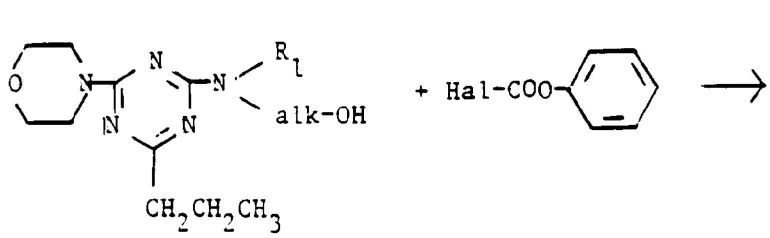

(XII)

(VIII) (XIII)

dans ces formules $R_1$ a la signification donnée ci-dessus, alk représente un radical alkylène en $C_1$-$C_4$ et Hal un atome d'halogène, de préférence un atome de chlore.

(g) Lorsque dans la formule I, $R_1$ représente le radical acétyle et $R_2$ un radical acétoxyalkyle dont le radical alkyle possède 1 à 4 atomes de carbone, on fait réagir au moins deux moles d'un halogénure d'acétyle, de préférence le chlorure d'acétyle, avec une mole d'un (4-morpholino-6-propyl-1,3,5-triazin-2-yl)aminoalcanol de formule XIV, selon l'équation

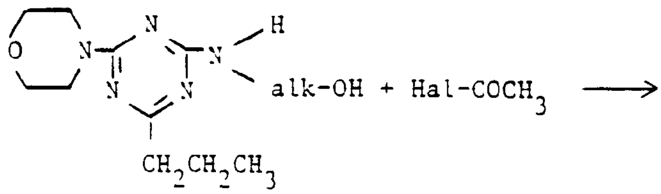


(I)

(XIV)

dans ces formules alk représente un radical alkylène en $C_1$-$C_4$ et Hal un atome d'halogène, de préférence un atome de chlore.

Cette réaction est effectuée dans un solvant inerte tel que le dichlorométhane, à une température comprise entre la température ambiante et la température de reflux du solvant, et en présence d'une base telle que la triéthylamine.

Les composés de départ de formule XIV peuvent être préparés selon l'un ou l'autre des deux procédés (a) ou (b) décrits précédemment.

Les sels d'addition d'acides non toxiques pharmaceutiquement acceptables peuvent être préparés à partir des 2-amino-4-morpholino-6-propyl-1,3,5-triazines de formule I par des méthodes connues en soi.

On donne ci-après à titre d'illustration la préparation de 2-amino-4-morpholino-6-propyl-1,3,5-triazines répondant à la formule générale I, à utiliser conformément à la présente invention.

- 2-[(4-morpholino-6-propyl-1,3,5-triazin-2-yl)amino]-éthanol (composé 1).

On dissout 145,5 g (0,6 mole) de 2-chloro-4-morpholino-6-propyl-1,3,5-triazine dans 600 ml de dioxanne. Dans cette solution on introduit, goutte à goutte et sous une bonne agitation, en 80 minutes environ, une solution de 444 g (7,28 moles) de 2-aminoéthanol dans 600 ml de dioxanne. Ensuite, le mélange réactionnel est chauffé à reflux pendant 7 heures. On refroidit, on décante le chlorhydrate de 2-aminoéthanol qui s'est formé et on élimine le dioxanne sous pression réduite. On dissout le résidu obtenu dans 5 litres d'éther diéthylique. Cette solution est lavée à l'eau et l'eau de lavage est réextraite par du dichlorométhane. Les phases organiques sont réunies et séchées sur sulfate de magnésium. On élimine le solvant sous pression réduite. Le résidu solide obtenu est recristallisé dans un mélange 50:50 (v/v) acétate d'éthyle-éther de pétrole. On obtient 139,8 g de 2-[(4-morpholino-6-propyl-1,3,5-triazin-2-yl)amino]-éthanol.
Rendement: 88%. P.F.: 95-99°C.

| Analyse pour $C_{12}H_{21}N_5O_2$ en % | | | |
|---|---|---|---|
| calculé: | C 53,91 | H 7,92 | N 26,20 |
| trouvé: | 54,30 | 8,10 | 26,13 |

Le 2-[(4-morpholino-6-propyl-1,3,5-triazin-2-yl)amino]-éthanol peut se présenter sous deux formes cristallines différentes selon que le produit a été recristallisé dans le méthanol (P.F.: 97-99°C) ou dans le toluène (P.F.: 89-90°C).

On prépare de la même manière les composés de formule I réunis dans le Tableau I:

## TABLEAU I

## 2-R-4-morpholino-6-propyl-1,3,5-triazines

| Composé | Substituant R | Mol (1) | Rdt (%) | P.F.(°C) | Analyses | calculé % | trouvé % |
|---|---|---|---|---|---|---|---|
| 2 | NHOH | 1,1 (2) | 51,6 | 93-94 | C | 55,68 | 55,70 |
| | | | | | H | 8,20 | 8,19 |
| | | | | | N | 32,24 | 32,19 |
| 3 | $NHCH_2CH_2CH_2OH$ | 2 | 86,6 | 95-96 | C | 55,51 | 55,02 |
| | | | | | H | 8,18 | 8,26 |
| | | | | | N | 24,91 | 24,42 |
| 4 | $NH-(CH_2)_3-CH_2OH$ | 2 | 74,6 | 79-80 | C | 56,95 | 56,95 |
| | | | | | H | 8,47 | 8,61 |
| | | | | | N | 23,73 | 23,42 |
| 5 | $NHCH_2-CH(OH)-CH_3$ | 3 | 90,0 | 104-105 | C | 55,52 | 55,50 |
| | | | | | H | 8,19 | 8,00 |
| | | | | | N | 24,91 | 23,48 |
| 6 | $NHCH_2-CH(OH)-C_2H_5$ | 1 (3) | 22,0 | 56-57 | C | 56,95 | 56,76 |
| | | | | | H | 8,47 | 8,37 |
| | | | | | N | 23,73 | 23,50 |
| 7 | $NH-CH(C_2H_5)-CH_2OH$ | 2 | 75,4 | 90-91 | C | 56,95 | 56,92 |
| | | | | | H | 8,47 | 8,40 |
| | | | | | N | 23,73 | 23,60 |

TABLEAU I (suite)

| Composé | Substituant R | Mol (1) | Rdt (%) | P.F.(°C) | Analyses | calculé % | trouvé % |
|---|---|---|---|---|---|---|---|
| 8 | $NH-C(CH_3)_2-CH_2OH$ | 2 | 32,2 | 85-86 | C | 56,95 | 57,87 |
| | | | | | H | 8,47 | 8,12 |
| | | | | | N | 23,73 | 24,24 |
| 9 | $N(CH_3)-CH_2CH_2OH$ | 2 | 62,6 | 32-33 | C | 55,51 | 55,48 |
| | | | | | H | 8,19 | 8,30 |
| | | | | | N | 24,91 | 24,86 |
| 10 | $NHCH_2CH_2OCH_3$ | 2 | 42,7 | 44-45 | C | 55,52 | 55,60 |
| | | | | | H | 8,19 | 8,20 |
| | | | | | N | 24,91 | 24,99 |
| 11 | $NHCH_2CHOHCH_2OH$ | 2 | 68,7 | 119-120 | C | 52,52 | 52,69 |
| | | | | | H | 7,74 | 7,76 |
| | | | | | N | 23,57 | 23,60 |
| 12 | $NH-C(CH_2OH)_3$ | 2 | 61,0 | 110-111 | C | 51,37 | 50,94 |
| | | | | | H | 7,64 | 7,53 |
| | | | | | N | 21,41 | 21,16 |
| 13 | $NH-C(CH_2OH)_2-CH_3$ | 2 | 48,0 | 121-122 | C | 54,02 | 54,13 |
| | | | | | H | 8,04 | 8,00 |
| | | | | | N | 22,5 | 22,2 |
| 14 | $N(CH_2C_6H_5)C_2H_4OH$ | 2 | 50 | 57-58 | C | 63,86 | 64,60 |
| | | | | | H | 7,56 | 7,76 |
| | | | | | N | 19,6 | 19,8 |
| 15 | $NHCH(CH_2OH)CHOHC_6H_5$ | 2 | 63 | 107-108 | C | 61,13 | 62,00 |
| | | | | | H | 7,24 | 7,45 |
| | | | | | N | 18,77 | 18,92 |
| 16(4) | $NHCH_2CHOHCH_3$ | 2 | 38 | 97-98 | C | 55,52 | 56,30 |
| | | | | | H | 8,19 | 8,42 |
| | | | | | N | 24,91 | 24,70 |

TABLEAU I (suite)

| Composé | Substituant R | Mol (1) | Rdt (%) | P.F.(°C) | Analyses | calculé % | trouvé % |
|---|---|---|---|---|---|---|---|
| 17(5) | $NHCH_2CHOHCH_3$ | 2 | 49 | 94-95 | C | 55,52 | 55,64 |
| | | | | | H | 8,19 | 8,13 |
| | | | | | N | 24,91 | 24,73 |
| 18 | 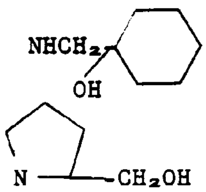 | 2 | 63 | 75-76 | C | 60,90 | 59,83 |
| | | | | | H | 8,66 | 8,73 |
| | | | | | N | 20,99 | 20,48 |
| 19(6) | | 2 | 47 | 134-136 | C | 52,40 | 52,48 |
| | | | | | H | 7,57 | 7,40 |
| | | | | | N | 20,38 | 19,83 |
| 20 | $NHN(CH_3)_2$ | 2 | 63,6 | 105-106 | C | 54,14 | 54,39 |
| | | | | | H | 8,97 | 8,81 |
| | | | | | N | 31,58 | 31,65 |

(1) nombre de moles de l'amine par mole de produit de départ;

(2) en présence de 2 moles de triéthylamine;

(3) en présence de 1 mole de triéthylamine;

(4) énantiomère de configuration S; $[\alpha]_D^{25}$ = +15,3 (C = 1, méthanol);

(5) énantiomère de configuration R; $[\alpha]_D^{25}$ = -16,1 (C = 1, méthanol);

(6) chlorhydrate.

La 2-chloro-4-morpholino-6-propyl-1,3,5-triazine utilisée comme produit de départ pour la synthèse des composés cités ci-dessus, a été préparée selon la méthode décrite par T. TSUJIKAWA et al., (Yakugaku Zasshi,95,(1975),512-520) à partir de la 2,4-dichloro-6-propyl-1,3,5-triazine. Ce dernier composé a été préparé selon le procédé de R. HIRT et al. (Helv.Chim.Acta.,33,(1950),1365-69).

- N-(4-morpholino-6-propyl-1,3,5-triazin-2-yl)-acétamide (composé 21).

A la température ambiante, on ajoute goutte à goutte 4 g (0,05 mole) de chlorure d'acétyle dans une solution de 11,2 g (0,05 mole) de 2-amino-4-morpholino-6-propyl-1,3,5-triazine dans 100 ml de pyridine anhydre. On agite le mélange pendant 6 heures puis on laisse reposer pendant 48 heures. On filtre le chlorhydrate de pyridine et on évapore le filtrat sous pression réduite. Le résidu obtenu est repris une fois par du toluène que l'on évapore à nouveau. Ensuite, on reprend le résidu par du dichlorométhane, on lave la solution à l'eau et on sèche sur sulfate de sodium. Le résidu obtenu après évaporation du solvant est purifié par chromatographie sur silice (éluant: 90:10 (v/v) dichlorométhane-éthanol) et le produit est finalement recristallisé dans l'acétate d'éthyle.

On obtient 5,75 g de N-(4-morpholino-6-propyl-1,3,5-triazin-2-yl)-acétamide. Rendement: 43,4%. P.F.: 141-142°C. (Composé 21a). Chlorhydrate: P.F.: 145-146°C (alcool isopropylique-éther). (Composé 21b).

Analyse pour $C_{12}H_{19}N_5O_2.HCl$ en %

| | | | | |
|---|---|---|---|---|
| calculé: | C 47,76 | H 6,63 | N 23,22 | $Cl^-$ 11,77 |
| trouvé: | 47,78 | 6,73 | 22,80 | 11,62 |

- N-(4-morpholino-6-propyl-1,3,5-triazin-2-yl)-benzamide (chlorhydrate) (composé 22).

Dans une solution de 11,2 g (0,05 mole) de 2-amino-4-morpholino-6-propyl-1,3,5-triazine dans 200 ml de dichloroéthane, on ajoute, à la température ambiante, successivement, une solution de 7,7 g (0,055 mole) de chlorure de benzoyle dans 50 ml de dichloroéthane et une solution de 5,5 g (0,055 mole) de triéthylamine dans 50 ml de dichloroéthane. On chauffe le mélange à reflux pendant 6 heures puis on le refroidit à la température ambiante. On lave successivement à l'eau, avec une solution aqueuse de bicarbonate de sodium, puis encore une fois à l'eau. On sèche la phase organique sur sulfate de sodium et on évapore le solvant sous pression réduite. Le résidu obtenu est chromatographié sur silice (éluant: 95:5 (v/v) dichlorométhane-éthanol) puis finalement recristallisé dans un mélange 50:50 (v/v) éther-hexane. Le produit forme un chlorhydrate dans l'éther. On obtient ainsi 12,1 g de chlorhydrate de N-(4-morpholino-6-propyl-1,3,5-triazin-2-yl)-benzamide. Rendement: 66,5%. P.F.: 197-198°C.

Analyse pour $C_{17}H_{21}N_5O_2.HCl$ en %

| | | | | |
|---|---|---|---|---|
| calculé: | C 56,12 | H 6,05 | N 19,26 | $Cl^-$ 9,77 |
| trouvé: | 56,30 | 6,50 | 19,16 | 9,54 |

Les composés réunis dans le tableau II ont été préparés, comme indiqué, selon la méthode qui a servi à préparer soit le composé 21, soit le composé 22.

TABLEAU II

2-R-4-morpholino-6-propyl-1,3,5-triazines

| Composé | Substituant R | Méthode du composé | Rdt (%) | P.F.(°C) | Analyses | calculé % | trouvé % |
|---|---|---|---|---|---|---|---|
| 23 | $NHCOCH_2C_6H_5$ | 21 | 51,3 | 110-113 | C | 63,34 | 63,18 |
| | | | | | H | 6,74 | 6,85 |
| | | | | | N | 20,52 | 20,55 |
| 24 | $NHCOCH(CH_3)_2$ | 21 | 34,3 | 141 | C | 57,34 | 56,84 |
| | | | | | H | 7,85 | 7,84 |
| | | | | | N | 23,89 | 23,70 |
| 25a | $N(CH_3)COCH_3$ | 21 | 34,8 | 60-61 | C | 55,91 | 56,02 |
| | | | | | H | 7,53 | 7,57 |
| | | | | | N | 25,09 | 25,05 |
| 25b(1) | $N(CH_3)COCH_3$ | 21 | 75,5 | 104-105 | C | 49,44 | 48,78 |
| | | | | | H | 6,97 | 7,01 |
| | | | | | N | 22,19 | 22,43 |
| 26 | $N(CH_3)COCH(CH_3)_2$ | 21 | 38,5 | 36-37 | C | 58,62 | 58,78 |
| | | | | | H | 8,16 | 8,33 |
| | | | | | N | 22,80 | 22,62 |
| 27(1) | $N(CH_3)COC_6H_5$ | 22 | 46,5 | 122 | C | 57,22 | 56,78 |
| | | | | | H | 6,36 | 6,40 |
| | | | | | N | 18,54 | 18,28 |
| 28 | $NH-COOC_6H_5$ | 22 | 24,8 | 154-155 | C | 59,48 | 59,60 |
| | | | | | H | 6,12 | 6,32 |
| | | | | | N | 20,41 | 20,31 |
| 29(1) | $NH-CO-C_6H_4-pCl$ | 22 | 66 | 205-206 | C | 51,27 | 51,19 |
| | | | | | H | 5,31 | 5,34 |
| | | | | | N | 17,58 | 17,49 |

TABLEAU II (suite)

| Composé | Substituant R | Méthode du composé | Rdt (%) | P.F.(°C) | Analyses calculé % | trouvé % |
|---|---|---|---|---|---|---|
| 30 | $N(CH_3)COC_6H_4$-pCl | 22 | 72 | 96-97 | C 57,52 | 58,07 |
| | | | | | H 5,90 | 6,17 |
| | | | | | N 18,63 | 18,65 |
| 31(1) | $NHCOC_6H_4$-p$OCH_3$ | 22 | 33 | 199 | C 54,89 | 54,83 |
| | | | | | H 6,14 | 6,17 |
| | | | | | N 17,78 | 17,80 |
| 32(1) | $N(CH_3)COC_6H_4$-p$OCH_3$ | 22 | 56 | 120 | C 55,94 | 55,40 |
| | | | | | H 6,42 | 6,48 |
| | | | | | N 17,17 | 17,22 |
| 33(1) | $NHCOC_6H_4$-p$CH_3$ | 22 | 53 | 188 | C 57,21 | 57,45 |
| | | | | | H 6,40 | 6,42 |
| | | | | | N 18,53 | 18,73 |
| 34(1) | $N(CH_3)COC_6H_4$-p$CH_3$ | 22 | 74 | 99 | C 58,23 | 58,08 |
| | | | | | H 6,69 | 6,73 |
| | | | | | N 17,87 | 18,11 |
| 35 | $N(CH_3)COC_6H_3$-(3,4-di-$OCH_3$) | 22 | 70 | 73 | C 59,84 | 60,12 |
| | | | | | H 6,78 | 7,09 |
| | | | | | N 17,44 | 17,45 |
| 36 | $NHCOC_6H_3$-(3,4-di-$CH_3$) | 22 | 45 | 232 | C 58,23 | 58,42 |
| | | | | | H 6,69 | 6,65 |
| | | | | | N 17,87 | 17,72 |

(1) chlorhydrate

- 2-[[2-(acétoxy)éthyl]amino]-4-morpholino-6-propyl-1,3,5-triazine (composé 37).

Dans une solution de 13,4 g (0,05 mole) de 2-[(4-morpholino-6-propyl-1,3,5-triazin-2-yl)amino]-éthanol (composé 1) dans 200 ml de dichlorométhane, on introduit simultanément, entre 0° et 5°C, 4,3 g (0,055 mole) de chlorure d'acétyle dissous dans 50 ml de dichlorométhane, et 5,5 g (0,055 mole) de triéthylamine en solution dans 50 ml de dichlorométhane. L'addition de ces deux réactifs est réglée de manière à ce que dans le milieu réactionnel, le chlorure d'acide soit toujours en excès par rapport à la triéthylamine. On maintient l'agitation du mélange pendant une heure à 5°C et ensuite pendant 12 heures à la température ambiante. On lave le mélange de réaction successivement avec une solution aqueuse de bicarbonate de sodium et avec de l'eau. On sèche sur sulfate de sodium et on évapore le solvant sous pression réduite. Le résidu cristallise dans un mélange

toluène-hexane 1:2. Le produit obtenu est d'abord purifié par chromatographie sur silice (éluant: 95:5 (v/v) dichlorométhane-éthanol) puis il est finalement recristallisé dans un mélange toluène-hexane 1:1. On obtient 9,3 g de 2-[[2-(acétoxy)éthyl]amino]-4-morpholino-6-propyl-1,3,5-triazine.
Rendement: 60%. P.F.: 94-95°C.

| Analyse pour $C_{14}H_{23}N_5O_3$ en % | | | |
|---|---|---|---|
| calculé: | C 54,37 | H 7,44 | N 22,65 |
| trouvé: | 54,98 | 7,69 | 22,62 |

On prépare de la même manière les composés réunis dans le tableau III.

TABLEAU III

| Composé | Substituant R | Rdt (%) | P.F.(°C) | Analyses calculé % | Analyses trouvé % |
|---|---|---|---|---|---|
| 38 | $NH(CH_2)_2O\text{-}COCH(CH_3)_2$ | 59,3 | 54-55 | C 56,97 | 56,86 |
| | | | | H 8,01 | 8,04 |
| | | | | N 20,77 | 20,74 |
| 39 | $NH(CH_2)_2O\text{-}COC_6H_5$ | 67,4 | 98-99 | C 61,46 | 61,52 |
| | | | | H 6,74 | 6,75 |
| | | | | N 18,87 | 18,91 |
| 40 | $NH(CH_2)_2O\text{-}COCH_2C_6H_5$ | 63,4 | 63-64 | C 62,34 | 62,45 |
| | | | | H 7,01 | 7,07 |
| | | | | N 18,18 | 18,15 |

- N,N-diméthyl-N'-(4-morpholino-6-propyl-1,3,5-triazin-2-yl)-urée (composé 41).

A la température de 20°C, on fait passer pendant trois heures un courant gazeux de diméthylamine dans une solution de 5 g (0,0145 mole) de (4-morpholino-6-propyl-1,3,5-triazin-2-yl)carbamate de phényle (composé 28) dans 100 ml de dichlorométhane anhydre. On laisse ensuite reposer le mélange pendant 12 heures. On le lave successivement avec une solution diluée d'hydroxyde de sodium et avec de l'eau. On sèche la phase organique sur sulfate de sodium et on évapore le solvant sous pression réduite. Le résidu obtenu cristallise dans un mélange 50:50 (v/v) acétate d'éthyle-hexane. On obtient 1,7 g de N,N-diméthyl-N'-(4-morpholino-6-propyl-1,3,5-triazin-2-yl)-urée.
Rendement: 39,9%. P.F.: 110-111°C.

| Analyse pour $C_{13}H_{22}N_6O_2$ en % | | | |
|---|---|---|---|
| calculé: | C 53,06 | H 7,48 | N 28,57 |
| trouvé: | 53,20 | 7,69 | 28,67 |

- 2-[[2-[(aminocarbonyl)oxy]éthyl]amino]-4-morpholino-6-propyl-1,3,5-triazine (composé 42).

Dans un litre d'ammoniac liquide, on introduit 19,4 g (0,05 mole) de 2-morpholino-4-[[2-[(phénoxycarbonyl)oxy]éthyl]amino]-6-propyl-1,3,5-triazine dissous dans 100 ml de dichlorométhane anhydre. On maintient l'agitation pendant 7 heures à une température comprise entre -35°C et -45°C. Ensuite, on évapore l'ammoniac

et on lave la phase organique successivement avec une solution aqueuse de bicarbonate de sodium et avec de l'eau. On sèche sur sulfate de sodium et on évapore le solvant sous pression réduite. Le résidu obtenu cristallise dans un mélange 50:50 (v/v) acétate d'éthyle-hexane. On obtient 15,1 g de 2-[[2-[(aminocarbonyl)oxy]éthyl]amino]-4-morpholino-6-propyl-1,3,5-triazine.
Rendement: 97,4%. P.F.: 139-140°C.

Analyse pour $C_{13}H_{22}N_6O_3$ en %

| | | |
|---|---|---|
| calculé: | C 50,32 | H 7,09 |
| trouvé: | 50,39 | 7,15 |

La 2-morpholino-4-[[2-[(phénoxycarbonyl)oxy]éthyl]amino]-6-propyl-1,3,5-triazine utilisée comme produit de départ dans cet exemple, a été préparée à partir de 2-[(4-morpholino-6-propyl-1,3,5-triazin-2-yl)amino]-éthanol et de chloroformiate de phényle, selon le procédé utilisé pour la synthèse du composé 37.
Rendement: 85%. P.F.: 83-84°C.

Analyse pour $C_{19}H_{25}N_5O_4$ en %

| | | | |
|---|---|---|---|
| calculé: | C 58,91 | H 6,46 | N 18,0 |
| trouvé: | 58,99 | 6,60 | 18,1 |

- N-[2-(acétoxy)éthyl]-N-(4-morpholino-6-propyl-1,3,5-triazin-2-yl)-acétamide (composé 43).

Dans une suspension de 13,4 g (0,05 mole) de 2-[(4-morpholino-6-propyl-1,3,5-triazin-2-yl)amino]-éthanol (composé 1) dans 250 ml de dichloroéthane, maintenue à une température voisine de 20°C, on ajoute une solution de 11,8 g (0,15 mole) de chlorure d'acétyle dans 50 ml de dichloroéthane et une solution de 15 g (0,15 mole) de triéthylamine dans 50 ml de dichloroéthane. Cette addition est effectuée de la manière suivante: on introduit d'abord la moitié du chlorure d'acide et on laisse réagir pendant 15 minutes, puis on introduit la moitié de la triéthylamine; ensuite, on introduit le restant du chlorure d'acide, on laisse encore réagir pendant 15 minutes, on chauffe le mélange à 55°C et finalement on introduit le restant de la triéthylamine. Le mélange obtenu est chauffé à reflux pendant 7 heures. Puis, on le laisse reposer pendant une nuit à la température ordinaire. On le lave successivement avec une solution aqueuse de bicarbonate de sodium et avec de l'eau. On sèche sur sulfate de sodium et on filtre en présence de norite. Après évaporation du solvant sous pression réduite, le résidu obtenu est purifié par chromatographie sur silice (éluant: 95:5 (v/v) dichlorométhane-éthanol). Le produit obtenu cristallise à froid dans l'hexane. On obtient 6,35 g de N-[2-(acétoxy)éthyl]-N-(4-morpholino-6-propyl-1,3,5-triazin-2-yl)-acétamide.
Rendement: 36,2%. P.F.: 49-50°C.

Analyse pour $C_{16}H_{25}N_5O_4$ en %

| | | | |
|---|---|---|---|
| calculé: | C 54,70 | H 7,12 | N 19,94 |
| trouvé: | 55,22 | 7,22 | 20,0 |

- 2-amino-4-morpholino-6-propyl-1,3,5-triazine (chlorhydrate) (composé 44).

Ce produit est décrit par S. MURAI et al. dans la demande de brevet japonaise 69.688/74 (Chem.Abstr.81,(1974),136188x). Il peut être préparé de la manière suivante.

On mélange 87 g (1 mole) de morpholine et 8,6 g (0,05 mole) de 2-amino-4-chloro-6-propyl-1,3,5-triazine. La température du mélange s'élève d'elle-même à 54°C. On chauffe ensuite à reflux pendant 5 heures. On évapore le mélange de réaction sous pression réduite et le résidu obtenu est redissous dans de l'acétate d'éthyle. On lave la solution trois fois à l'eau, puis on la sèche sur sulfate de sodium. Le solvant est éliminé sous pression réduite et le résidu est recristallisé dans de l'hexane. On obtient 9,1 g de 2-amino-4-morpholino-6-propyl-1,3,5-triazine.
Rendement: 82%. P.F.: 127-128°C.
Chlorhydrate: P.F.: 210-211°C (alcool isopropylique-éther).

Analyse pour $C_{10}H_{17}N_5O.HCl$ en %

| | | | | |
|---|---|---|---|---|
| calculé: | C 46,24 | H 6,94 | N 26,97 | Cl- 13,6 |
| trouvé: | 46,21 | 6,90 | 26,78 | 13,6 |

La 2-amino-4-chloro-6-propyl-1,3,5-triazine utilisée comme produit de départ dans la synthèse du composé 44 a été préparée selon la méthode de S. MURAI et al. (demande de brevet japonaise 69.688/74).

- 2-(méthylamino)-4-morpholino-6-propyl-1,3,5-triazine (chlorhydrate) (composé 45).

A une solution contenant 5,6 g (0,03 mole) de 2-chloro-4-(méthylamino)-6-propyl-1,3,5-triazine dans 50 ml de dioxanne, on ajoute une solution contenant 8,7 g (0,1 mole) de morpholine dans 50 ml de dioxanne. Le mélange est chauffé à reflux pendant 5 heures. Ensuite, on refroidit et on filtre le chlorhydrate de morpholine qui s'est formé. Le solvant est éliminé sous pression réduite et le résidu est repris dans du chloroforme. On lave à l'eau et on sèche la phase organique sur sulfate de sodium. On évapore le solvant et le résidu est recristallisé dans l'acétate d'éthyle. On obtient 5,3 g de 2-(méthylamino)-4-morpholino-6-propyl-1,3,5-triazine. Rendement: 74%. P.F.: 131-133°C.

Le produit obtenu est dissous dans de l'alcool isopropylique à chaud. A cette solution, on ajoute la quantité équivalente d'acide chlorhydrique en solution dans de l'éther éthylique. Le chlorhydrate cristallise par refroidissement. On filtre, on lave à l'éther éthylique et on sèche.
Rendement: 75%. P.F.: 177-178°C.

Analyse pour $C_{11}H_{19}N_5O.HCl$ en %

| | | | | |
|---|---|---|---|---|
| calculé: | C 48,26 | H 7,31 | N 25,59 | Cl- 12,97 |
| trouvé: | 48,38 | 7,40 | 25,57 | 12,64 |

La 2-chloro-4-(méthylamino)-6-propyl-1,3,5-triazine utilisée comme produit de départ dans la synthèse du composé 45 a été préparée selon la méthode de T. TSUJIKAWA et al., (Yakugaku Zasshi,95,(1975),512-520).

Comme il a été indiqué plus haut, les 2-amino-4-morpholino-6-propyl-1,3,5-triazines de formule I, ainsi que leurs sels d'addition d'acides non toxiques pharmaceutiquement acceptables à utiliser conformément à l'invention, possèdent la propriété de corriger les effets d'un hypofonctionnement du système cholinergique. Dans les exemples qui suivent, cette propriété avantageuse est mise en évidence par une série d'essais pharmacologiques par lesquels on montre que les composés de formule I présentent certains effets similaires à ceux de composés cholinergiques bien connus tels que l'oxotrémorine, l'arécoline ou la physostigmine, ou bien, s'opposent à l'effet d'un antagoniste cholinergique comme la scopolamine.

Exemple 1. Potentialisation des effets cholinergiques de l'oxotrémorine.

Ce test (R.C. RATHBUN et al., Psychopharmacologia,4,(1963),114-125) a pour but de montrer que les composés de formule I potentialisent des effets cholinergiques centraux et périphériques provoqués par l'administration chez la souris d'une faible dose d'oxotrémorine;

Le degré d'activation cholinergique périphérique est mesuré par l'effet sialagogue quantifié par le système de cotation suivant:

score 0: la salive n'excède pas celle émise par une souris normale;
score 1: un peu de salive se trouve autour des dents;
score 2: la salive dessine une bande étroite autour de la bouche;
score 4: la salive mouille une surface sous le menton;
score 6: la salive coule de la bouche par exemple sur les membres antérieurs.

Les scores intermédiaires ne sont pas employés et la salive est enlevée après chaque observation.

Le degré d'activation cholinergique centrale est mesuré par l'effet trémogène quantifié par le système de cotation suivant:

score 0: pas de tremblement;
score 1: tremblements occasionnels légers et périodiques;
score 2: tremblements modérés et légers se répétant souvent;
score 4: tremblements accentués, mais entrecoupés de périodes de calme;

score 6: tremblements très accentués et quasi-continuels.

Des souris mâles NMRI (20 à 25 g) sont réparties en quatre groupes de cinq animaux, à savoir: un groupe témoin et trois groupes traités.

Le composé à tester est administré par voie intrapéritonéale (aux trois groupes traités) 20 minutes avant l'administration de l'oxotrémorine, à une dose différente pour chacun des groupes traités.

L'oxotrémorine est administrée par voie intrapéritonéale aux groupes traités et au groupe témoin, à la dose de 0,05 mg/kg, en solution dans 10 ml/kg de sérum physiologique. Cette dose correspond environ à la dose minimale d'oxotrémorine qui fait apparaître des tremblements et de la salivation.

Après l'administration de l'oxotrémorine, les animaux sont placés individuellement dans de petites cages et sont observés à intervalles réguliers de 5 minutes jusqu'à disparition complète des effets cholinergiques.

Pour chacun des groupes, les scores individuels relevés à chaque période d'observation sont additionnés; ceci permet d'établir une courbe représentant la somme des scores en fonction du temps, caractéristique de chaque groupe.

Les valeurs moyennes des surfaces sous les courbes obtenues pour chacun des groupes traités sont comparées à la valeur moyenne de la surface sous la courbe correspondant au groupe témoin et font l'objet d'une analyse statistique selon la méthode de Mann-Whitney. A partir de cette comparaison, on peut déterminer une "dose minimale active". Cette "dose minimale active" est la dose minimale de composé nécessaire pour observer encore une potentialisation de l'effet sialagogue ou trémogène de l'oxotrémorine, ou autrement dit, pour obtenir une surface plus grande que la surface sous la courbe obtenue pour le groupe témoin.

Les résultats obtenus à ce test ont montré que les composés de formule I potentialisent l'effet sialagogue de l'oxotrémorine à une dose minimale comprise entre 7,7 et 107 mg/kg, et potentialisent l'effet trémogène à une dose minimale comprise entre 8 et 112 mg/kg. De plus, les doses minimales actives ne présentent pas d'effets cholinergiques propres et sont très éloignées des doses mortelles déterminées au test d'Irwin.

Par ailleurs, ce test a mis en évidence que certains composés de formule I, administrés à la dose minimale active, ont des effets de plus longue durée que l'ester méthylique de l'acide 1,2,5,6-tétrahydro-1-méthyl-3-pyridinecarboxylique (arécoline), composé cholinergique bien connu.

Ainsi, les composés de formule I montrent une potentialisation des effets sialagogues et trémogènes de l'oxotrémorine vingt minutes après avoir été administrés à la dose minimale active, alors que, dans les mêmes conditions, l'arécoline ne donne aucune potentialisation.

Exemple 2. Inhibition de l'hyperactivité induite par la scopolamine.

Un animal placé pour la première fois dans un environnement nouveau manifeste une intense activité exploratrice de ce nouveau milieu. La diminution progressive et ensuite la disparition de cette activité exploratrice, en d'autres termes, l'habituation au nouveau milieu, peut être considérée comme une forme élémentaire d'apprentissage. Cette forme élémentaire d'apprentissage est sensible à l'influence des médicaments qui facilitent ou qui ralentissent l'apprentissage (A. PLATEL et al., Psychopharmacology,78,(1982),346-352). Ainsi, par exemple, la scopolamine, composé qui provoque des troubles mnésiques, induit une hyperactivité exploratrice chez le rat placé dans un environnement nouveau; ce phénomène est lié à l'activité anticholinergique centrale de ce composé (W.J. STEWART et al., Psychopharmacologia,44,(1975), 291-295). Par contre, un agoniste cholinergique comme la physostigmine s'oppose à l'hyperactivité induite par la scopolamine.

Dans le test décrit ci-après, selon leur solubilitié, les composés de formule I sont administrés soit dans du sérum physiologique, soit dans un véhicule approprié (généralement un tampon citrate à pH 5). Ce test permet d'établir pour les composés de formule I une activité comparable à celle de la physostigmine.

Il est basé sur la technique originale décrite par A. PLATEL (loc.cit.), d'une part, et d'autre part sur le procédé d'automatisation de l'enregistrement des mesures décrit par H.J. TAUGER et al., (J.Neuroscience Methods,10,(1984),237-245).

Dans ce test, on utilise des rats mâles Sprague-Dawley SPF (Specific Pathogen Free; 160 à 200 g). Pendant la semaine qui précède l'expérience, ces animaux sont gardés dans des conditions normales par groupes de 15 animaux dans des cages grillagées standards, et la nourriture et la boisson sont librement accessibles.

Au début de l'expérience, les rats sont répartis en 4 groupes homogènes de 10 animaux et sont habitués pendant une heure au local d'expérimentation. Ensuite, chaque groupe d'animaux est soumis à un traitement déterminé:

– le groupe 1 reçoit deux injections intrapéritonéales simultanées de sérum physiologique ou de véhicule utilisé;

– le groupe 2 reçoit une injection intrapéritonéale de sérum physiologique ou du véhicule utilisé et une injection intrapéritonéale du 0,5 mg/kg de scopolamine en solution;

– le groupe 3 reçoit une injection intrapéritonéale du composé à tester en solution dans un véhicule approprié et
une injection intrapéritonéale de sérum physiologique ou du véhicule utilisé;
– le groupe 4 reçoit une injection intrapéritonéale du composé à tester et
une injection intrapéritonéale de 0,5 mg/kg de scopolamine.

Trente minutes après ce traitement, les quatre groupes d'animaux sont testés simultanément. A cet effet, chaque groupe est réparti dans une enceinte carrée (100 x 100 cm) constituée d'un sol grillagé et d'une paroi verticale de 50 cm de hauteur. Chacune de ces enceintes contient 16 zones comprenant 4 zones dans les coins, 4 zones centrales et 8 zones périphériques. En outre, chaque enceinte est équipée de 2 rangées de cellules à infra-rouge disposées à 2 cm au-dessus du sol afin d'enregistrer les déplacements horizontaux des animaux et de 2 autres rangées de cellules à infra-rouge disposées à une hauteur de 10 cm du sol pour l'enregistrement des déplacements verticaux. Ces cellules à infra-rouge sont reliées à un microprocesseur qui permet la détermination de la moyenne des distances parcourues (en cm) par un groupe d'animaux, la détermination du nombre moyen de redressements effectués, de même que la répartition des déplacements horizontaux dans les différentes zones centrales, périphériques et des coins, exprimés par le temps moyen de séjour dans ces diverses zones.

Chaque composé testé est étudié au moins à trois doses différentes à partir desquelles on détermine la dose minimale active qui inhibe l'hyperactivité induite par l'administration intrapéritonéale de 0,5 mg/kg de scopolamine. On compare ensuite soit les valeurs obtenues dans le groupe 2 traité seulement à la scopolamine avec celles qui sont obtenues dans le groupe 4 traité à la fois par la scopolamine et par le composé à tester, soit les valeurs obtenues dans le groupe 4 avec celles obtenues dans le groupe 3 traité seulement par le composé à tester. Le groupe témoin 1 sert de contrôle pour l'action de la scopolamine administrée au groupe 2. Les différences observées sont évaluées statistiquement dans tous les cas par la méthode de Mann-Whitney.

Les résultats obtenus dans ce test ont montré que les composés de formule I possèdent une bonne activité inhibitrice de l'hyperactivité induite par la scopolamine. Les doses minimales actives déterminées pour ces composés sont comprises entre 1 et 89 mg/kg. Mais, contrairement à la physostigmine qui est inactive lorsqu'elle est administrée plus de 15 minutes avant d'effectuer les mesures, les composés de formule I possèdent encore une activité inhibitrice lorsqu'ils sont administrés 30 minutes avant d'effectuer les mesures. Leur activité est donc de plus longue durée que celle de la physostigmine.

Exemple 3. Inhibition des effets de la scopolamine sur l'électroencéphalogramme. (EEG).

L'administration de scopolamine à l'homme ou à l'animal induit des troubles mnésiques comparables à ceux qui apparaissent au cours du vieillissement normal ou pathologique. Chez des patients souffrant de démence sénile de type Alzheimer, on a obtenu une amélioration des troubles mnésiques par l'administration de physostigmine, composé qui inhibe l'acétyl-cholinestérase.

La scopolamine, administrée par voie intrapéritonéale à des rats, à la dose de 0,5 mg/kg, induit des variations du spectre EEG, qui se traduisent par une augmentation de la puissance de la bande de 8 Hz, une diminution de la puissance des bandes de 20,8 à 40 Hz ainsi qu'une augmentation de la puissance totale du spectre EEG. La physostigmine s'oppose à ces variations.

L'objectif de ce test est de montrer, au moyen de la méthode d'analyse quantitative de l'électroencéphalogramme, que les composés de formule I ont la propriété de neutraliser les effets que la scopolamine exerce sur le spectre EEG (P. ETEVENON, L'Electroencéphalographie sur ordinateur. Analyse quantitative et statistique. Copedith, Paris, 1978).

Dans ce test, on utilise des rats mâles Albinos Sprague-Dawley SPF (160 à 200 g).

Lorsque les animaux sont âgés de 3 mois, on implante à demeure, aseptiquement et sous anesthésie générale, 5 électrodes corticales: une électrode indifférente, une électrode frontale gauche et une droite, et une électrode occipitale gauche et une droite (P. ETEVENON, loc. cit.).

Le test est effectué lorsque les animaux sont âgés d'environ 15 mois. Dans l'intervalle, les animaux sont maintenus dans des cages individuelles. Ils reçoivent de l'eau et de la nourriture à volonté et subissent un cycle journalier régulier comportant une période d'obscurité entre 18 heures du soir et 6 heures du matin. En même temps, les animaux sont progressivement habitués aux cages de la cabine insonorisée qu'ils occuperont ultérieurement pour l'enregistrement des électroencéphalogrammes, de même qu'aux conditions expérimentales, par l'administration de placébo par voie intrapéritonéale. Les produits sont administrés immédiatement avant l'enregistrement de l'EEG.

Les rats sont répartis par groupe de 8 animaux et on enregistre 16 échantillons de spectres EEG en 5 secondes (deux spectres EEG par animal). Les spectres obtenus sont ensuite analysés par ordinateur (transformée de Fourier rapide), ce qui permet de déterminer pour chaque groupe la moyenne des 16 mesures effectuées et d'en déduire, pour un animal, la puissance totale du spectre EEG ainsi que la distribution de cette

puissance (en %) dans les différentes bandes de fréquences.

Cette opération (enregistrement des spectres) est répétée 9 fois (durée totale: 121 minutes).

L'effet du composé étudié est déduit de la comparaison statistique des résultats obtenus pour les différents groupes d'animaux auxquels ont été administrés respectivement le composé à tester, la scopolamine et un placébo.

Dans le tableau IV ci-dessous, on donne pour quelques composés de formule I, administrés par voie intrapéritonéale à la dose indiquée en mg/kg, le pourcentage de réduction de l'augmentation de la puissance de la bande 6,4 à 9,6 Hz (moyenne 8 Hz), augmentation provoquée par l'administration intrapéritonéale de 0,5 mg/kg de scopolamine.

TABLEAU IV

Inhibition des effets de la scopolamine sur la bande de 6,4 à 9,6 Hz de l'EEG.

| Composé | Dose (mg/kg) | Inhibition (en %) |
|---|---|---|
| 1 | 5,3 | 52 |
| 7 | 29,5 | 50 |
| 9 | 8,8 | 53,3 |
| 20 | 2,7 | 66,7 |
| 21a | 2,7 | 100 |
| 22 | 3,6 | 66,7 |
| 25a | 0,88 | 46,7 |
| 26 | 3,1 | 55,6 |
| 27 | 12 | 93,8 |
| 37 | 9,9 | 53,3 |

TABLEAU IV (suite)

| Composé | Dose (mg/kg) | Inhibition (en %) |
|---|---|---|
| 43 | 11,2 | 77,8 |
| 44 | 0,8 | 100 |
| 45 | 2,7 | 91,7 |
| physostigmine | 0,4 | 76,8 |

Les résultats montrent que les composés de formule I, comme la physostigmine, inhibent les effets que la scopolamine exerce sur l'électroencéphalogramme. On voit que cette inhibition atteint et dépasse même 50% à des doses relativement faibles, très éloignées des doses toxiques, ce qui n'est pas le cas pour la physostigmine.

Exemple 4. Evitement passif à essais multiples.

Les composés de formule I ont été étudiés en vue de faire apparaître d'une part, leur propriété de favoriser l'apprentissage exprimée par la réduction du nombre d'essais nécessaires pour atteindre un critère prédéterminé, et d'autre part, leur propriété de s'opposer à l'amnésie provoquée par l'administration de scopolamine.

A cet effet, on a utilisé la méthode de l'évitement passif à essais multiples. Cette méthode est bien connue pour évaluer les effets qu'un produit exerce sur la mémoire et l'apprentissage (A. FINE et al., Proc. Natl.Acad.Sci.USA,82,(1985),5227-5230).

Le test est pratiqué sur des rats mâles Sprague-Dawley (160-200 g) qui sont maintenus, pendant l'expérience, dans des cages standards.

L'appareil utilisé est une cage transparente carrée, de 35 cm de côté et de 25 cm de hauteur, équipée d'un plancher grillagé électrifiable. Un tapis isolant (10 x 17 cm) en caoutchouc est placé sur le sol dans un des coins de la cage.

Pour évaluer si un composé est susceptible de favoriser l'apprentissage, on effectue l'essai suivant.

Chaque animal est placé sur le tapis en caoutchouc et on note le temps que l'animal met pour se décider à quitter cet emplacement pour explorer la cage. Après 20 secondes d'exploration, l'animal reçoit un choc électrique (durée 3 secondes) dans les pattes, provoquant une réaction de fuite. Le rat est immédiatement retiré de l'appareil et replacé dans sa cage d'origine. On répète cette expérience jusqu'à ce que l'animal reste au moins 180 secondes sur le tapis de caoutchouc pour éviter le choc électrique. L'apprentissage est exprimé par le nombre moyen d'essais nécessaires pour atteindre un temps de séjour de 180 secondes sur le tapis.

Un temps de séjour de 180 secondes sur le tapis de caoutchouc est considéré comme étant la performance maximum à réaliser par l'animal pour éviter le choc électrique. Les rats qui séjournent pendant ce temps sur le tapis ont acquis le réflexe d'évitement et sont replacés dans leur cage d'origine sans recevoir de choc électrique.

Pour évaluer si un composé est susceptible de favoriser la rétention mnésique au cours du temps, on effectue l'expérience suivante. Chaque animal est soumis à quatre essais aux temps 0, 4, 24 et 28 heures. Au premier essai (temps 0), l'animal est placé sur le tapis en caoutchouc et on note le temps qu'il met pour se décider à quitter cet emplacement pour explorer la cage. Après 20 secondes d'exploration, le rat reçoit un choc électrique (durée 3 secondes) dans les pattes, provoquant une réaction de fuite. Le rat est immédiatement retiré de l'appareil et replacé dans sa cage d'origine. Au cours des trois essais ultérieurs (temps: 4, 24 et 28 heures), l'animal est replacé sur le tapis en caoutchouc et l'on note le temps mis pour quitter cet emplacement. Dès que les quatre pattes de l'animal reposent sur la grille, il reçoit un choc électrique et il est immédiatement retiré de l'appareil.

Au début de l'expérience, les rats sont répartis en 4 groupes homogènes de 15 animaux. Trente minutes avant chaque essai, chaque groupe d'animaux est soumis à un traitement déterminé:

– le groupe 1 reçoit une injection intrapéritonéale de sérum physiologique;
– le groupe 2 reçoit une injection intrapéritonéale du composé à tester;
– le groupe 3 reçoit une injection intrapéritonéale de 0,5 mg de scopolamine et
– le groupe 4 reçoit une injection intrapéritonéale de 0,5 mg de scopolamine et une injection intrapéritonéale du composé à tester, simultanément.

Les groupes 1 et 2 sont utilisés dans la première expérience et les groupes 3 et 4 dans la seconde expérience.

Les résultats obtenus dans ce test avec les composés de formule I sont repris dans le tableau V. Dans ce tableau, on indique le numéro du composé soumis à l'essai (colonne 1) et la dose administrée par voie intrapéritonéale, exprimée en mg/kg (colonne 2).

Dans les colonnes 3 et 4, on donne les résultats obtenus dans l'essai utilisé pour évaluer l'apprentissage. Les chiffres indiquent le nombre moyen d'essais nécessaires pour qu'un animal témoin (groupe 1) ou traité (groupe 2) par le composé apprenne à séjourner pendant 180 secondes sur le tapis de caoutchouc pour éviter le choc électrique. Les résultats ont été analysés par le test de Student.

Dans les colonnes 5 à 12, on donne les résultats obtenus dans l'expérience utilisée pour évaluer la rétention mnésique. Dans les colonnes 5 à 8, les chiffres représentent les temps de séjour moyens observés respectivement aux temps 0, 4, 24 et 28 heures pour les animaux du groupe 3, traités seulement à la scopolamine, et dans les colonnes 9 à 12, on trouve les chiffres correspondants pour les animaux du groupe 4, traités simultanément par la scopolamine et par le composé étudié (à la dose indiquée dans la deuxième colonne).

L'influence favorable d'un composé pour s'opposer à l'amnésie provoquée par la scopolamine est démontrée par l'augmentation du temps de séjour sur le tapis, à chaque observation. Les différences observées sont analysées statistiquement par la méthode de Mann-Whitney.

TABLEAU V

| Composé | Dose (mg/kg) | Apprentissage nombre moyen d'essais groupe 1 | Apprentissage nombre moyen d'essais groupe 2 | Rétention mnésique Temps de séjour (en secondes) groupe 3: 0 | groupe 3: 4 | groupe 3: 24 | groupe 3: 28 | groupe 4: 0 | groupe 4: 4 | groupe 4: 24 | groupe 4: 28 |
|---|---|---|---|---|---|---|---|---|---|---|---|
| 1 | 5,35 | 2,27 | 1,53 | 1,27 | 13,3 | 24,2 | 26,3 | 2,7 | 44,8 | 61,3 | 102,7 |
| 7 | 29,54 | 2,67 | 2,57 | 1,0 | 1,5 | 3,3 | 4,0 | 1,3 | 7,3 | 22,5 | 52,1 |
| 9 | 8,72 | 2,27 | 2,0 | 1,4 | 2,3 | 3,3 | 5,7 | 3,3 | 67,9 | 62,7 | 160,3 |
| 10 | 28,14 | 2,87 | 2,07 | 1,8 | 4,5 | 2,3 | 4,5 | 5,5 | 15,7 | 31,1 | 34,1 |
| 20 | 2,66 | 1,93 | 1,53 | 1,1 | 2,5 | 4,6 | 5,5 | 2,7 | 55,3 | 57,7 | 87,9 |
| 21a | 2,65 | 1,60 | 1,53 | 2,4 | 9,7 | 9,1 | 33,9 | 3,4 | 60,4 | 74,6 | 102,4 |
| 22 | 3,64 | 2,20 | 1,0 | 1,7 | 11,1 | 20,3 | 12,8 | 2,8 | 70,9 | 82,8 | 137,6 |
| 25a | 0,89 | 2,07 | 2,07 | 2,1 | 3,0 | 7,7 | 3,3 | 1,7 | 8,1 | 34,7 | 44,5 |
| 27 | 3,78 | 2,20 | 1,13 | 1,9 | 8,9 | 31,6 | 54,1 | 11,3 | 88,6 | 111,5 | 133,5 |
| 37 | 9,6 | 2,0 | 1,3 | 1,0 | 2,5 | 4,5 | 5,8 | 2,9 | 24,0 | 44,4 | 49,9 |
| 41 | 9,12 | 2,53 | 1,33 | 1,0 | 3,7 | 7,0 | 7,7 | 1,3 | 15,8 | 27,3 | 66,3 |
| 44 | 2,6 | 1,73 | 1,13 | 2,0 | 16,3 | 29,8 | 33,0 | 2,6 | 25,7 | 43,6 | 58,3 |
| 45 | 2,74 | 1,60 | 2,00 | 1,0 | 10,9 | 8,3 | 8,1 | 4,5 | 94,7 | 77,5 | 99,1 |
| physostigmine | 0,4 | 2,60 | 2,30 | 1,7 | 1,4 | 6,9 | 21,3 | 1,9 | 17,8 | 56,2 | 53,4 |

De ce tableau, il apparaît que

– les composés de formule I favorisent l'apprentissage du réflexe d'évitement: le nombre moyen d'essais nécessaires pour atteindre le critère prédéterminé (temps de séjour maximum de 180 secondes sur le tapis) est moins élevé pour les animaux traités (colonne 4) que pour les animaux témoins (colonne 3);

– l'effet amnésiant de la scopolamine est très marqué: on voit que les temps de séjour des animaux du groupe 3 (colonnes 5 à 8) sont nettement moindres que les 180 secondes réalisées par les témoins après un nombre moyen d'essais (colonne 3); et dans ces conditions l'influence favorable des composés de formule I pour s'opposer à l'effet amnésiant de la scopolamine est très nette; les animaux du groupe 4, traités simultanément par la scopolamine et par un composé de formule I, ont des temps de séjour, à chaque observation, considérablement plus élevés que les animaux du groupe 3 traités à la scopolamine seule (comparer les résultats de la colonne 5 avec ceux de la colonne 9, 6 avec 10).

– la physostigmine exerce une action favorable contre l'effet amnésiant de la scopolamine, similaire à celle des composés de formule I utilisés selon l'invention, mais elle est obtenue à une dose présentant des effets secondaires et très proche de la dose toxique, ce qui n'est pas le cas pour les composés de formule I.

Exemple 5. Toxicité.

La toxicité des composés de formule I a été déterminée chez la souris mâle NMRI au moyen du test d'Irwin (S. IRWIN, Psychopharmacologia,13,(1968),222-257).

Des doses progressives du composé à tester sont administrées par voie intrapéritonéale à des groupes de trois souris jusqu'à ce que la dose mortelle soit atteinte (dose provoquant la mort de deux animaux sur trois dans les 48 heures).

Dans le tableau VI ci-dessous, on donne la dose mortelle observée pour les composés de formule I à utiliser conformément à l'invention. Il ressort de ce tableau que ces composés sont très peu toxiques, contrairement à la physostigmine.

TABLEAU VI

| Composé | Dose mortelle (en mg/kg) | Composé | Dose mortelle (en mg/kg) |
|---|---|---|---|
| 1 | 802 | 24 | 880 |
| 2 | 718 | 25a | 838 |
| 3 | 844 | 25b | 947 |
| 4 | 886 | 26 | 922 |
| 5 | 563 | 27 | 1133 |
| 6 | 886 | 28 | > 1030 |
| 7 | 886 | 29 | 398 |
| 8 | 886 | 30 | 1125 |
| 9 | 844 | 31 | 511 |
| 10 | 844 | 32 | 1222 |
| 11 | 892 | 33 | 1132 |
| 12 | 982 | 34 | 1174 |
| 13 | 934 | 35 | 401 |
| 14 | > 1073 | 36 | 1174 |
| 15 | 1121 | 37 | 928 |
| 16 | 844 | 38 | > 1012 |
| 17 | 844 | 39 | > 1114 |
| 18 | 1007 | 40 | 1157 |
| 19 | 1031 | 41 | 294 |
| 20 | 799 | 42 | > 931 |
| 21a | 996 | 43 | 351 |
| 21b | 905 | 44 | 779 |
| 22 | 1091 | 45 | 821 |
| 23 | > 1024 | physostigmine | 0,82 |

Exemple 6. Posologie et administration.

Les compositions pharmaceutiques renfermant les composés de formule I peuvent être administrées par voie orale, parentérale ou rectale. Les compositions pharmaceutiques utilisables pour l'administration orale peuvent être solides ou liquides, par exemple sous forme de comprimés (enrobés ou non), pilules, dragées, capsules en gélatine, solutions, sirops. Elles comprennent également les compositions qui permettent de délivrer la substance active d'une manière progressive. De même, les compositions utilisables pour l'administration par voie parentérale, sont les formes pharmaceutiquement connues pour ce genre d'administration, par exemple des solutions, des suspensions ou émulsions aqueuses ou huileuses.

Pour l'administration par voie rectale, les compositions contenant les composés à utiliser selon l'invention se présentent généralement sous forme de suppositoires.

Les formes pharmaceutiques telles que solutions injectables, suspensions injectables, comprimés, gouttes, suppositoires, etc. sont préparées selon les méthodes couramment utilisées par les pharmaciens.

On mélange les composés de formule I avec un véhicule solide ou liquide, non toxique, pharmaceutiquement acceptable et, éventuellement avec un agent dispersant, un agent désintégrant, un agent émulsionnant, un agent stabilisant. On peut y ajouter, le cas échéant, des agents édulcorants, des agents colorants, des

agents lubrifiants et des additifs divers.

Le pourcentage de substance active dans les compositions pharmaceutiques peut varier dans des limites très larges de manière à ce que le composé thérapeutique actif soit présent en quantité suffisante pour administrer les doses situées dans l'intervalle spécifié.

Quant à la posologie journalière, elle peut varier dans une gamme étendue de doses unitaires, par exemple de 0,1 à 2 g de produit actif selon le patient, le mode d'administration et en particulier selon la fréquence d'administration.

En général, il s'est avéré avantageux d'administrer, sous forme de comprimés, des quantités de 0,25 à 0,75 g, de préférence 0,5 g, une à trois fois par jour.

On donne ci-après, à titre d'illustration, quelques exemples de compositions pharmaceutiques contenant un composé de formule I.

1. Comprimés pour administration par voie orale.

| | |
|---|---|
| Composé 1 | 250 mg |
| Méthylcellulose (Methocel K4M®) | 200 mg |
| Lactose sec | 154 mg |
| Aerosil® | 5 mg |
| Acide citrique anhydre | 60 mg |
| Talc | 11 mg |
| Stéarate de magnésium | 20 mg |

2. Capsules de gélatine molle.

Pour environ 10.000 capsules contenant 50 mg de substance active, on prépare une solution de composition suivante:

| | |
|---|---|
| Composé 44 | 500 g |
| Polyéthylèneglycol 400 | 3300 g |
| Glycérine | 250 g |
| Eau | 1400 g |
| | 5450 g |

La solution est introduite de manière connue en soi dans des capsules de gélatine molle de dimension adéquate. Les capsules doivent être avalées.

3. Gouttes.

Pour des gouttes dosées à 5 mg/ml, on prépare la solution suivante:

| | |
|---|---|
| Composé 45 | 5 g |
| Ethanol à 96% | 450 g |
| Méthylparaben | 1 g |
| Polyéthylèneglycol 400 | 50 g |
| Sirop de sucre à 50% | 400 g |
| Arôme et colorant alimentaire | 0,5 g |
| Eau | complément à 1000 ml |

On dissout le composé 45, le méthylparaben, l'arôme et le colorant dans l'éthanol à température ambiante. On ajoute ensuite le polyéthylèneglycol 400 et le sirop de sucre sous agitation et on complète par de l'eau jusqu'à obtention d'un volume de 1000 ml.

On introduit la solution dans des flacons de verre brun et, le cas échéant, on peut y ajouter des édulcorants.

## Revendications

1. Utilisation d'une 2-amino-4-morpholino-6-propyl-1,3,5-triazine répondant à la formule

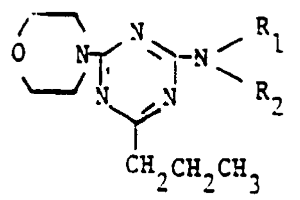

(I)

dans laquelle

$R_1$ représente un atome d'hydrogène, un radical alkyle, aralkyle ou acétyle,

$R_2$ représente un atome d'hydrogène, un groupe hydroxyle, un radical alkyle, hydroxyalkyle, alkoxyalkyle, dialkylamino, aryl-hydroxyalkyle, (hydroxy-cycloalkyl)alkyle, alkanoyloxyalkyle, benzoyloxyalkyle, phénylacétyloxyalkyle, aminocarbonyloxyalkyle, un groupe $COR_3$ dans lequel $R_3$ représente un radical alkyle, aryle, halogénoaryle, alkylaryle, alkoxyaryle, aralkyle ou aryloxy, ou un groupe $CONR_4R_5$ dans lequel $R_4$ et $R_5$ représentent de l'hydrogène ou un radical alkyle, ou

$R_1$ et $R_2$ forment conjointement avec l'atome d'azote auquel ils sont attachés un radical alkylèneimino substitué par un radical hydroxyalkyle, les radicaux alkyle, alkoxy et alkanoyloxy ayant de 1 à 4 atomes de carbone et les radicaux cycloalkyle et alkylèneimino ayant de 4 à 6 atomes de carbone,

avec la restriction que lorsque $R_1$ représente le radical acétyle,

$R_2$ représente un radical acétoxyalkyle, ou d'un de ses sels d'addition d'acides non toxiques pharmaceutiquement acceptables pour la préparation de médicaments destinés au traitement des troubles cognitifs associés à l'âge et aux syndromes démentiels.

2. Utilisation selon la revendication 1, caractérisée en ce qu'on utilise la 2-amino-4-morpholino-6-propyl-1,3,5-triazine ou un de ses sels non toxiques pharmaceutiquement acceptables.

3. Utilisation selon la revendication 1, caractérisée en ce qu'on utilise la 2-(méthylamino)-4-morpholino-6-propyl-1,3,5-triazine ou un de ses sels non toxiques pharmaceutiquement acceptables.

4. Utilisation selon la revendication 1, caractérisée en ce qu'on utilise le 2-[(4-morpholino-6-propyl-1,3,5-triazin-2-yl)amino]-éthanol ou un de ses sels non toxiques pharmaceutiquement acceptables.

5. Utilisation selon la revendication 1, caractérisée en ce qu'on utilise la 2-(hydroxyamino)-4-morpholino-6-propyl-1,3,5-triazine ou un de ses sels non toxiques pharmaceutiquement acceptables.

6. Utilisation selon la revendication 1, caractérisée en ce qu' on utilise la 2-[(2-méthoxyéthyl)amino]-4-morpholino-6-propyl-1,3,5-triazine ou un de ses sels non toxiques pharmaceutiquement acceptables.

7. Utilisation selon la revendication 1, caractérisée en ce qu'on utilise le (S)-3-[(4-morpholino-6-propyl-1,3,5-triazin-2-yl)amino]-2-propanol ou un de ses sels non toxiques pharmaceutiquement acceptables.

8. Utilisation selon la revendication 1, caractérisée en ce qu'on utilise le (R)-3-[(4-morpholino-6-propyl-1,3,5-triazin-2-yl)amino]-2-propanol ou un de ses sels non toxiques pharmaceutiquement acceptables.

9. Utilisation selon la revendication 1, caractérisée en ce qu'on utilise la 2-(2,2-diméthylhydrazino)-4-morpholino-6-propyl-1,3,5-triazine ou un de ses sels non toxiques pharmaceutiquement acceptables.

10. Utilisation selon la revendication 1, caractérisée en ce qu'on utilise le N-(4-morpholino-6-propyl-1,3,5-triazin-2-yl)-benzamide ou un de ses sels non toxiques pharmaceutiquement acceptables.

11. Utilisation selon la revendication 1, caractérisée en ce qu'on utilise le N-méthyl-N-(4-morpholino-6-propyl-1,3,5-triazin-2-yl)-benzamide ou un de ses sels non toxiques pharmaceutiquement acceptables.

12. Utilisation selon la revendication 1, caractérisée en ce qu'on utilise la 2-[[2-(acétoxy)éthyl]amino]-4-morpholino-6-propyl-1,3,5-triazine ou un de ses sels non toxiques pharmaceutiquement acceptables.

## Claims

1. Use of a 2-amino-4-morpholino-6-propyl-1,3,5-triazine of the formula

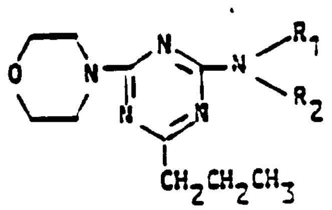

(I)

wherein

$R_1$ represents a hydrogen atom, an alkyl, aralkyl or acetyl radical,

$R_2$ represents a hydrogen atom, a hydroxyl group, an alkyl, hydroxyalkyl, alkoxyalkyl, dialkylamino, arylhydroxyalkyl, (hydroxy-cycloalkyl)alkyl, alkanoyloxyalkyl, benzoyloxyalkyl, phenylacetyloxyalkyl or aminocarbonyloxyalkyl radical, a $COR_3$ group, in which $R_3$ represents an alkyl, aryl, haloaryl, alkylaryl, alkoxyaryl, aralkyl or aryloxy radical, or a $CONR_4R_5$ group, in which $R_4$ and $R_5$ represent a hydrogen atom or an alkyl radical, or

$R_1$ and $R_2$, together with the nitrogen atom to which they are attached, form an alkyleneimino radical substituted by a hydroxyalkyl radical, the alkyl, alkoxy and alkanoyloxy radicals having 1 to 4 carbon atoms and the cycloalkyl and alkyleneimino radicals having 4 to 6 carbon atoms,

with the proviso that when $R_1$ represents the acetyl radical, $R_2$ represents an acetoxyalkyl radical,

or one of its non-toxic pharmaceutically acceptable acid addition salts for the preparation of medicaments for the treatment of cognitive disorders associated with aging and with dementia syndromes.

2. Use according to claim 1, characterised in that use is made of 2-amino-4-morpholino-6-propyl-1,3,5-triazine or one of its non-toxic pharmaceutically acceptable acid addition salts.

3. Use according to claim 1, characterised in that use is made of 2-(methylamino)-4-morpholino-6-propyl-1,3,5-triazine or one of its non-toxic pharmaceutically acceptable acid addition salts.

4. Use according to claim 1, characterised in that use is made of 2-[(4-morpholino-6-propyl-1,3,5-triazin-2-yl)amino]-ethanol or one of its non-toxic pharmaceutically acceptable acid addition salts.

5. Use according to claim 1, characterised in that use is made of 2-(hydroxyamino)-4-morpholino-6-propyl-1,3,5-triazine or one of its non-toxic pharmaceutically acceptable acid addition salts.

6. Use according to claim 1, characterised in that use is made of 2-[(2-methoxyethyl)amino]-4-morpholino-6-propyl-1,3,5-triazine or one of its non-toxic pharmaceutically acceptable acid addition salts.

7. Use according to claim 1, characterised in that use is made of (S)-3-[(4-morpholino-6-propyl-1,3,5-triazin-2-yl)amino]-2-propanol or one of its non-toxic pharmaceutically acceptable acid addition salts.

8. Use according to claim 1, characterised in that use is made of (R)-3-[(4-morpholino-6-propyl-1,3,5-triazin-2-yl)amino]-2-propanol or one of its non-toxic pharmaceutically acceptable acid addition salts.

9. Use according to claim 1, characterised in that use is made of 2-(2,2-dimethylhydrazino)-4-morpholino-6-propyl-1,3,5-triazine or one of its non-toxic pharmaceutically acceptable acid addition salts.

10. Use according to claim 1, characterised in that use is made of N-(4-morpholino-6-propyl-1,3,5-triazin-2-yl)-benzamide or one of its non-toxic pharmaceutically acceptable acid addition salts.

11. Use according to claim 1, characterised in that use is made of N-methyl-N-(4-morpholino-6-propyl-1,3,5-triazin-2-yl)-benzamide or one of its non-toxic pharmaceutically acceptable acid addition salts.

12. Use according to claim 1, characterised in that use is made of 2-[[2-(acetoxy)ethyl]amino]-4-morpholino-6-propyl-1,3,5-triazine or one of its non-toxic pharmaceutically acceptable acid addition salts.

## Patentansprüche

1. Verwendung von einem 2-Amino-4-morpholino-6-propyl-1,3,5-triazin der allgemeinen formel

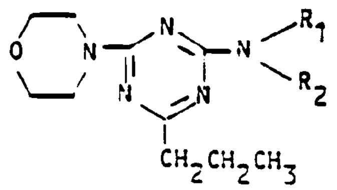

(I)

worin

$R_1$ ein Wasserstoffatom, einen Alkyl-, Aralkyl- oder Acetylrest darstellt,

$R_2$ ein Wasserstoffatom, eine Hydroxylgruppe, einen Alkyl-, Hydroxyalkyl-, Alkoxyalkyl-, Dialkylamino-, Aryl-hydroxyalkyl-, (Hydroxycycloalkyl)alkyl-, Alkanoyloxyalkyl-, Benzoyloxyalkyl-, Phenylacetyloxyalkyl- oder Aminocarbonyloxyalkylrest, eine Gruppe $COR_3$, in der $R_3$ einen Alkyl-, Aryl-, Halogenaryl-, Alkylaryl-, Alkoxyaryl-, Aralkyl- oder Aryloxyrest darstellt, oder eine Gruppe $CONR_4R_5$, in der $R_4$ und $R_5$ Wasserstoff oder einen Alkylrest bedeuten, darstellt, oder

$R_1$ und $R_2$ zusammen mit dem Stickstoffatom, an das sie gebunden sind, einen durch einen Hydroxyalkylrest substituierten Alkyleniminorest bilden, wobei die Alkyl-, Alkoxy- und Alkanoyloxyreste 1 bis 4 Kohlenstoffatome und die Cycloalkyl- und Alkyleniminoreste 4 bis 6 Kohlenstoffatome aufweisen, mit der Maßgabe, daß wenn $R_1$ den Acetylrest darstellt, $R_2$ einen Acetoxyalkylrest darstellt,

oder von einem seiner Additions-Salze nicht-toxischer pharmazeutisch annehmbarer Saüren zur Herstellung von Arzneimitteln zur Behandlung von Gedächtnisstörungen, die in Verbindung mit dem Alter und Demenz-Syndromen auftreten.

2. Verwendung nach Anspruch 1, dadurch gekennzeichnet, daß man das 2-Amino-4-morpholino-6-propyl-1,3,5-triazin oder ein seiner Additions-Salze nicht-toxischer pharmazeutisch annehmbarer Säuren verwendet.

3. Verwendung nach Anspruch 1, dadurch gekennzeichnet, daß man das 2-(Methylamino)-4-morpholino-6-propyl-1,3,5-triazin oder ein seiner Additions-Salze nicht-toxischer pharmazeutisch annehmbarer Säuren verwendet.

4. Verwendung nach Anspruch 1, dadurch gekennzeichnet, daß man das 2-[(4-Morpholino-6-propyl-1,3,5-triazin-2-yl)amino]-ethanol oder ein seiner Additions-Salze nicht-toxischer pharmazeutisch annehmbarer Säuren verwendet.

5. Verwendung nach Anspruch 1, dadurch gekennzeichnet, daß man das 2-(Hydroxyamino)-4-morpholino-6-propyl-1,3,5-triazin oder ein seiner Additions-Salze nicht-toxischer pharmazeutisch annehmbarer Säuren verwendet.

6. Verwendung nach Anspruch 1, dadurch gekennzeichnet, daß man das 2-[(2-Methoxyethyl)amino]-4-morpholino-6-propyl-1,3,5-triazin oder ein seiner Additions-Salze nicht-toxischer pharmazeutisch annehmbarer Säuren verwendet.

7. Verwendung nach Anspruch 1, dadurch gekennzeichnet, daß man das (S)-3-[(4-Morpholino-6-propyl-1,3,5-triazin-2-yl)amino]-2-propanol oder ein seiner Additions-Salze nicht-toxischer pharmazeutisch annehmbarer Säuren verwendet.

8. Verwendung nach Anspruch 1, dadurch gekennzeichnet, daß man das (R)-3-[(4-Morpholino-6-propyl-1,3,5-triazin-2-yl)amino]-2-propanol oder ein seiner Additions-Salze nicht-toxischer pharmazeutisch annehmbarer Säuren verwendet.

9. Verwendung nach Anspruch 1, dadurch gekennzeichnet, daß man das 2-(2,2-Dimethylhydrazino)-4-morpholino-6-propyl-1,3,5-triazin oder ein seiner Additions-Salze nicht-toxischer pharmazeutisch annehmbarer Säuren verwendet.

10. Verwendung nach Anspruch 1, dadurch gekennzeichnet, daß man das N-(4-Morpholino-6-propyl-1,3,5-triazin-2-yl)-benzamid oder ein seiner Additions-Salze nicht-toxischer pharmazeutisch annehmbarer Säuren verwendet.

11. Verwendung nach Anspruch 1, dadurch gekennzeichnet, daß man das N-Methyl-N-(4-morpholino-6-propyl-1,3,5-triazin-2-yl)-benzamid oder ein seiner Additions-Salze nicht-toxischer pharmazeutisch annehmbarer Säuren verwendet.

12. Verwendung nach Anspruch 1, dadurch gekennzeichnet, daß man das 2-[[2-(Acetoxy)ethyl]amino]-4-morpholino-6-propyl-1,3,5-triazin oder ein seiner Additions-Salze nicht-toxischer pharmazeutisch annehmbarer Säuren verwendet.